# EUROPÄISCHE PATENTSCHRIFT

(11) **EP 1 539 368 B1**
(45) Veröffentlichungstag und Bekanntmachung des Hinweises auf die Patenterteilung: **08.12.2010**
(21) Anmeldenummer: 03794928.6
(22) Anmeldetag: 23.08.2003
(51) Int. Cl.: B05B 11/00, A61M 15/00, A61M 5/30, A61M 5/50

(54) **Gerät mit Blockiervorrichtung für ein Sperrspannwerk mit federbetätigtem Antrieb**
Apparatus with blocking device for a sprung lock with a spring mechanism
Appareil avec dispositif de blocage pour un mécanisme tendeur à blocage pourvu d'un mécanisme à ressort

(30) Priorität: 28.08.2002 DE 10239443
(43) Veröffentlichungstag der Anmeldung: 15.06.2005
(73) Patentinhaber: Boehringer Ingelheim International GmbH, 55216 Ingelheim am Rhein (DE)
(72) Erfinder: SCHYRA, Michael, 42111 Wuppertal (DE); WACHTEL, Herbert, 55218 Ingelheim (DE)
(86) Internationale Anmeldenummer: PCT/EP2003/009364
(87) Internationale Veröffentlichungsnummer: WO 2004/024340

(56) Entgegenhaltungen:
- WO-A-93/21980
- WO-A-95/34874
- WO-A-97/12687
- WO-A-97/24586

## Beschreibung

Die Erfindung betrifft eine Blockiervorrichtung, mit der der bestimmungsgemäße Gebrauch eines Gerätes, das mit einem Sperrspannwerk und federbetätigtem Abtrieb ausgerüstet ist, nach einer vorgegebenen Anzahl von Betätigungen verhindert wird. Bei dem Gerät kann es sich zum Beispiel um einen Hochdruckzerstäuber oder um einen nadellosen Injektor handeln.
Die Erfindung bezweckt, die Gebrauchsdauer eines derartigen Gerätes zuverlässig zu begrenzen und Sicherheitsforderungen zu erfüllen. Die Gründe für die Begrenzung können beispielsweise hygienischer, medizinischer oder technischer Art sein.

Das Sperrspannwerk, das nach Ablauf der zulässigen Gebrauchsdauer blockiert werden soll, umfasst bevorzugt ein - in einem von Hand zu betätigenden Gerät untergebrachtes - Schraub-Schub-Getriebe, mit dem eine Drehbewegung in eine lineare Bewegung umgesetzt und eine Arbeitsfeder gespannt wird. Die Arbeitsfeder wirkt auf ein Sprungstück des Sperrspannwerks, dessen Bewegung zunächst gesperrt wird, sobald die Arbeitsfeder den gespannten Zustand erreicht hat. In dem Sprungstück kann ein Kolben befestigt sein, der in einem Zylinder verschiebbar gelagert ist. Innerhalb des Zylinders befindet sich vor dem Kolben eine Flüssigkeit, die beim Auslösen der Sperrvorrichtung des Sperrspannwerks mittels des von der Arbeitsfeder angetriebenen Kolbens durch eine Düse hindurch ausgestossen wird. Die Anzahl der Betätigungen des Sperrspannwerks und damit des Gerätes kann durch ein mechanisches Zählwerk gezählt werden.

In WO-93/21980 ist ein Dosier-Inhalator angegeben. Die zu inhalierende Dosis einer Substanz wird durch eine von Hand betätigte Vorrichtung aus einem im Inhalator vorhandenen Vorrat der Substanz in eine Kammer eingebracht, aus der die Dosis mit dem Luftstrom ausgetragen wird, den der Benutzer beim Einatmen durch den Inhalator hindurch ansaugt. Der Dosier-Inhalator ist mit einer Zählvorrichtung versehen, die eine drehbare Schraubenspindel und einen Stab umfasst, dessen eines Ende in Form einer Nase in das Gewinde der Schraubenspindel eingreift. Der Stab verschiebt sich parallel zur Schraubenspindel mit zunehmender Drehung der Spindel. Das Zählwerk zeigt mittels der Position des nasenförmigen Endes des Stabes die Anzahl der Teilmengen an, die aus dem Substanzvorrat bereits entnommen worden sind, oder die daraus noch entnehmbar sind. Das andere Ende des Stabes wird in einem Führungsschacht verschiebbar gehalten, in den der Stab mit zunehmender Drehung der Schraubenspindel weiter eintaucht. Sobald der Substanzvorrat im Inhalator zur Neige geht, erreicht das nasenförmige Ende des Stabes, das in die Schraubenspindel eingreift, den Teil der Spindel, der mit einigen Gewindegängen versehen ist, deren Steigung grösser ist als im übrigen Teil der Schraubenspindel. Dadurch verschiebt sich der Stab bei jeder Drehung der Schraubenspindel schneller als vorher. Das andere Ende des Stabes drückt dabei auf einen biegsamen Hebel, und die weitere Betätigung des Dosier-Inhalators wird verhindert.

In WO-97/20590 ist ein Sperrspannwerk für einen federbetätigten Abtrieb angegeben. In WO-97/24586 wird ein mechanisches Zählwerk für ein Dosiergerät beschrieben. In WO-97/12687 ist eine Vorrichtung zur Hochdruckerzeugung in einem Fluid in Miniaturausführung angegeben, die mit einem Sperrspannwerk und einem Zählwerk versehen ist. Die Vorrichtung dient zum Zerstäuben eines Fluids zu einem inhalierbaren Aerosol. In WO-01/64268 wird ein nadelloser Injektor beschrieben, der ein Sperrspannwerk enthält.

Die beispielhaft genannten Geräte sind für wiederholte Benutzung vorgesehen, zum Beispiel für das wiederholte Zerstäuben einer vorgelegten Flüssigkeitsmenge zu einem lungengängigen Aerosol, oder zum nadellosen Injizieren einer vorgelegten Flüssigkeitsmenge unter die menschliche oder tierische Haut. Die zerstäubte oder injizierte Flüssigkeitsmenge kann eine therapeutisch wirksame Substanz enthalten.

Die Aufgabe der vorliegenden Erfindung ist, eine Vorrichtung für ein Gerät anzugeben, die nach einer vorgegebenen Anzahl von Betätigungen die weitere Benutzung des Gerätes zuverlässig, wirksam und endgültig verhindert, falls dafür ein triftiger Grund vorliegt. Das Gerät umfasst ein Sperrspannwerk mit einer Arbeitsfeder und einem Sprungstück, in dem ein Kolben angebracht ist, der in einem Zylinder verschiebbar gelagert ist. Die Bauteile sind in einem zweiteiligen Gehäuse untergebracht, das ein Gehäuseoberteil und ein Gehäuseunterteil umfasst. Beide Gehäuseteile sind gegeneinander drehbar gelagert. Die Arbeitsfeder wird mittels eines Schraub-Schub-Getriebes durch Drehen der beiden Gehäuseteile gegeneinander von Hand gespannt. Gleichzeitig mit dem Drehen der Gehäuseteile gegeneinander wird ein mechanisches Zählwerk betätigt, das eine Gewindespindel und einen Reiter umfasst. Die Gewindespindel ist in der Wand des unteren Gehäuseteils angebracht. Der Reiter wird um ein von der Anzahl der Drehungen der beiden Gehäuseteile gegeneinander abhängiges Stück auf der Spindel nach oben oder nach unten verschoben.

Diese Aufgabe wird erfindungsgemäß gelöst durch ein Gerät mit Blockiervorrichtung nach Anspruch 1.

Der Stössel kann einerseits an dem Reiter seitlich zur Spindel des Zählwerks angebracht sein. Bei dieser Ausführung der Blockiervorrichtung bewegt sich der Reiter bei bestimmungsgemässem Gebrauch des Gerätes in Richtung zum oberen Spindellager und zum oberen Gehäuseteil. Die Aussparung in der Wand des unteren Gehäuseteils ist neben der Achse der Zählwerksspindel angebracht. Bevor der Reiter am oberen Spindellager anstösst, verschiebt der Stössel das in der Aussparung in der Wand des unteren Gehäuseteils liegende Blockierelement aus seiner Ruheposition und aktiviert damit die Blockiervorrichtung. Der Stössel kann andererseits als eine Verlängerung des Blockierelements ausgebildet sein. Bei dieser Ausführung ragt das Ende des Stössels in den Weg, den der Reiter bei bestimmungsgemässem Gebrauch des Gerätes zurücklegt, bevor der Reiter am oberen Lager der Zählwerksspindel anliegt. Diese Ausführung wirkt genau so wie die vorstehend beschriebene Ausführung.

Bei einer weiteren Ausführung der Blockiervorrichtung kann der Stössel als Verlängerung der Zählwerksspindel ausgeführt sein und über das obere Spindellager hinausragen. In diesem Fall ist die Zählwerksspindel axial verschiebbar gelagert. Die Aussparung in der Wand des unteren Gehäuseteils ist bevorzugt in der Achse der Zählwerksspindel angebracht. Vor dem Aktivieren der Blockiervorrichtung wird die Zählwerksspindel durch eine Feder, zum Beispiel eine Schraubenfeder, gegen das untere Spindellager gedrückt. Bei dieser Ausführung der Blockiervorrichtung bewegt sich der Reiter bei bestimmungsgemässem Gebrauch des Gerätes in Richtung zum unteren Spindellager. Sobald der Reiter am unteren Spindellager anliegt, verschiebt sich die Zählwerksspindel bei ihrer weiteren Drehung in axialer Richtung zum oberen Gehäuseteil. Die als Stössel ausgebildete Verlängerung der Zählwerksspindel verschiebt das in der Aussparung in der Wand des unteren Gehäuseteils liegende Blockierelement aus dessen Ruheposition und aktiviert damit die Blockiervorrichtung.
In Umkehrung der beschriebenen Ausführungen kann das Blockierelement aus seiner Ruheposition mittel des Reiters herausgezogen werden.

Das in der Wand des unteren Gehäuseteils vorhandene Blockierelement kann in axialer oder in radialer Richtung bewegbar sein. Das Blockierelement kann eine Blattfeder sein, bevorzugt eine vorgespannte Blattfeder mit zwei Schenkeln, die bevorzugt aus Metall besteht.

Die erfindungsgemäße Blockiervorrichtung hat folgende Vorteile:
- Sie ist für miniaturisierte Geräte geeignet.
- Sie ist zwischen den einander überlappenden Gehäuseteilen angeordnet und bei einem Gerät im Gebrauchszustand für den Benutzer nicht mehr zugänglich.
- Sie ist einfach zu montieren.
- Ein Blockierelement in Form einer vorgespannten Blattfeder mit zwei Schenkeln ist in ihrer Ruheposition ohne zusätzlichen Aufwand gegen Verschieben gesichert.
- Eine vorgespannte Blattfeder lässt sich mit einer relativ geringen Kraft aus ihrer Ruheposition hinausschieben oder herausziehen.
- Eine vorgespannte Blattfeder mit zwei Schenkeln springt schlagartig von ihrer Ruheposition in ihre Position bei aktivierter Blockiervorrichtung, sobald sie mittels eines Stössels um eine vorgegebene Strecke bewegt worden ist. Damit ist der Ansprechpunkt der Blockiervorrichtung genau festgelegt.
- Das Drehen beider Gehäuseteile gegeneinander wird unmittelbar blockiert, sobald das ursprünglich in der Aussparung in der Wand des unteren Gehäuseteils vorhandene Blockierelement in beiden Aussparungen gleichzeitig liegt.
- Die aktivierte Blockiervorrichtung, die eine vorgespannte metallene Blattfeder enthält, läßt sich nur durch ein mehrere Newtonmeter grosses Kraftmoment überwinden, mit dem das blockierte Gerät zerstört wird.

Die erfindungsgemäße Blockiervorrichtung wird beispielsweise in einem HochdruckZerstäuber oder in einem nadellosen Injektor benutzt. Eine mit einem derartigen Gerät verabreichte medizinische Flüssigkeit kann ein in einem Lösemittel gelöstes Arzneimittel enthalten. Als Lösemittel sind beispielsweise Wasser, Ethanol oder deren Mischungen geeignet. Als Arzneimittel werden beispielsweise Berotec (Fenoterol-Hydrobromid; 1-(3,5-dihydroxy-phenyl)-2-[[1-(4-hydroxy-benzyl)-ethyl]-amino]-ethanol-hydrobromid), Atrovent (Ipratropiumbromid), Berodual (Kombination aus Fenoterol-Hydrobromid und Ipratropiumbromid), Salbutamol (oder Albuterol), Combivent, Oxivent (Oxitropium- bromid), Ba 679 (Tiotropiumbromid), BEA 2108 (Di-(2-thienyl)glykolsäuretropenol- ester), Flunisolid, Budesonid und andere verwendet.

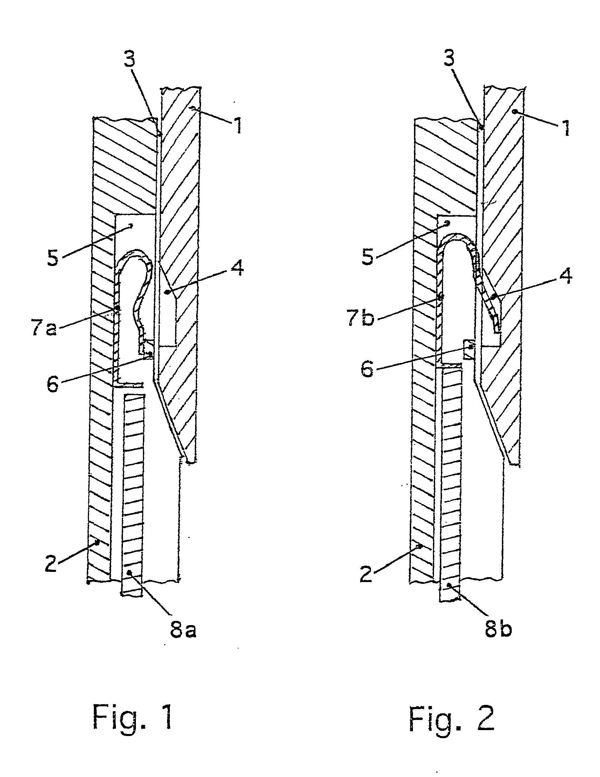

Eine bevorzugte Ausführungsform der erfindungsgemässen Blockiervorrichtung wird an Hand der Figuren weiter erläutert. Die Figuren 1 und 2 zeigen ausschnittsweise jeweils einen Längsschnitt durch die Wand des unteren und des oberen Gehäuseteils sowie einer Blattfeder als Blockierelement und eines Stössels in Höhe der Aussparungen in den Wänden. Der Längsschnitt liegt parallel zur Achse des unteren und des oberen Gehäuseteils.

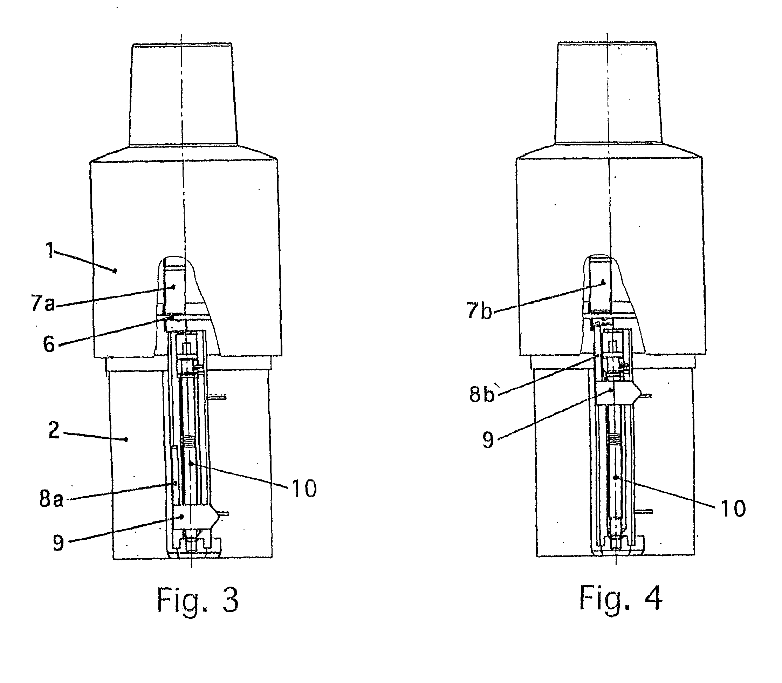

Die Figuren 3 und 4 zeigen die Längsansicht eines Gerätes, das im Bereich des Zählwerks und der Blockiervorrichtung aufgeschnitten ist.

In den Figuren 1 und 3 ist die Blockiervorrichtung im Ruhezustand dargestellt. Das Blockierelement befindet sich in seiner Ruheposition und nur in der Aussparung in der Wand des unteren Gehäuseteils. In den Figuren 2 und 4 ist die aktiviert Blockiervorrichtung dargestellt. Das Blockierelement ist aus seiner Ruheposition verschoben und befindet sich in beiden Aussparungen in den Wänden der beiden Gehäuseteile.
Das obere Gehäuseteil (1) überlappt das untere Gehäuseteil (2). Der Spalt (3) zwischen den beiden gegeneinander drehbar gelagerten Gehäuseteilen ist überhöht dargestellt. Im oberen Gehäuseteil befindet sich die Aussparung (4) und im unteren Gehäueteil die Aussparung (5). Die Aussparung (5) enthält einen hinterschnittenen Vorsprung (6), der an den Seitenwänden der Aussparung (5) mit dem unteren Gehäuseteil verbunden ist. Der Stössel (8a, 8b) ragt in die Aussparung in der Wand des unteren Gehäuseteils hinein. Der Stössel ist an dem (in den Figuren 1 und 2 nicht dargestellten) Reiter angebracht, der sich auf der (in den Figuren 1 und 2 nicht dargestellten) Spindel des Zählwerks befindet. Das Blockierelement ist eine Blattfeder mit zwei Schenkeln. Die Blattfeder (7a) ist in ihrer Ruheposition zwischen dem Boden der Aussparung (5) und dem hinterschnittenen Vorsprung (6) eingeklemmt. Der Stössel (8a) ist in einer Position dargestellt, die er kurz vor dem Berühren der Blattfeder (7a) erreicht hat. Bei der weiteren Betätigung des Gerätes bewegt sich der Stössel in Richtung auf die Blattfeder und verschiebt diese aus ihrer Ruheposition, bis das Ende der Blattfeder hinter dem Vorsprung (6) hervorspringt. Falls in diesem Augenblick beide Aussparungen (4) und (5) noch nicht einander gegenüber liegen, stösst das hinter dem Vorsprung (6) hervorgesprungene Ende der Blattfeder zunächst an die Innenwand des oberen Gehäuseteils. Sobald bei weiterem Drehen der beiden Gehäuseteile gegeneinander beide Aussparungen (4) und (5) einander gegenüber liegen, springt das Ende der Blattfeder in die Aussparung (4). Damit liegt die Blattfeder (7b) in beiden Aussparungen, und die Blockiervorrichtung ist aktiviert.
In der Figur 3 ist die Spindel (10) des Zählwerkes dargestellt, auf der sich der Reiter (9) in seiner (unteren) Ausgangsposition befindet, bevor das Gerät zum ersten Mal benutzt wird. Der Stössel (8a) ist von der Blattfeder (7a) weit entfernt.
In der Figur 4 befindet sich der Reiter auf der Spindel in seiner (oberen) Endposition, in der er das eine Ende der Blattfeder (8b) berührt und die Blattfeder aus ihrer Ruheposition herausgeschoben hat, wodurch die Blockiervorrichtung aktiviert worden ist.

Die in den Figuren dargestellte vorgespannte Blattfeder (7a; 7b) als Blockierelement besteht zum Beispiel aus Federstahlblech mit etwa 0,2 mm Dicke und ist etwa 3,5 mm breit. Die beiden Aussparungen in den Wänden der beiden Gehäuseteile sind etwa 4 mm breit und jeweils etwa 1 mm tief. Die beiden Gehäuseteile können bei aktivierter Blockiervorrichtung nur mit grossem Kraftaufwand (Kraftmoment etwa 3 Newtonmeter) gegeneinander gedreht werden, wobei jedoch das Gerät selbst zerstört und unbrauchbar wird.

## Patentansprüche

1. Gerät (1) mit Blockiervorrichtung, das ein Sperrspannwerk mit einer Arbeitsfeder und einem Sprungstück umfasst, in dem ein Kolben angebracht ist, der in einem Zylinder verschiebbar gelagert ist, und diese Bauteile in einem zweiteiligen Gehäuse untergebracht sind, das ein Gehäuseoberteil (1) und ein Gehäuseunterteil (2) umfasst, und beide Teile gegeneinander drehbar gelagert sind, und die Arbeitsfeder mittels eines Schraub-Schub-Getriebes durch Drehen der beiden Gehäuseteile gegeneinander von Hand gespannt wird, und gleichzeitig mit dem Drehen der Gehäuseteile gegeneinander ein mechanisches Zählwerk betätigt wird, das eine Gewindespindel (10) und einen Reiter (9) umfasst, und die Gewindespindel in der Wand des unteren Gehäuseteils angebracht ist, und der Reiter um ein von der Anzahl der Drehungen der beiden Gehäuseteile gegeneinander abhängiges Stück auf der Spindel verschoben wird, wobei
• in der Außenwand des unteren Gehäuseteils und in der Innenwand des oberen Gehäuseteils jeweils eine Aussparung (4, 5) vorhanden ist, und
• beide Aussparungen bei einer vorgegebenen Drehstellung beider Gehäuseteile einander gegenüber liegen, und
• ein bewegbares Blockierelement (7a, 7b) vorhanden ist, das sich zunächst nur in der Aussparung im unteren Gehäuseteil befindet, und
• ein Stössel zum Bewegen des Blockierelements vorhanden ist, der mit dem Reiter (9) auf der Spindel (10) des Zählwerks zusammenwirkt.

2. Gerät nach Anspruch 1, wobei
• der Stössel (8a, 8b) am Reiter (9) angebracht ist.

3. Gerät nach Anspruch 1, wobei
• der Stössel (8a, 8b) am Blockierelement (7a, 7b) angebracht ist.

4. Gerät nach Anspruch 1, wobei
• der Stössel (8a, 8b) als Verlängerung der Spindel (10) des Zählwerks ausgebildet ist und die Spindel (10) axial verschiebbar gelagert ist.

5. Gerät nach Anspruch 1, wobei
• das Blockierelement in axialer Richtung bewegbar ist.

6. Gerät nach Anspruch 1, wobei
• das Blockierelement eine vorgespannte Blattfeder (7a,7b) ist.

7. Gerät nach den Ansprüchen 1 und 6, wobei
• das Blockierelement eine vorgespannte Blattfeder (7a, 7b) mit zwei Schenkeln ist und bevorzugt aus Metall besteht.

8. Verwendung des Geräts nach Anspruch 1 zum Blockieren eines Hochdruck-Zerstäubers in Miniaturausführung zum Zerstäuben einer Flüssigkeit, die einen pharmazeutischen Wirkstoff enthält, zu einem Aerosol.

9. Verwendung des Geräts nach Anspruch 1 zum Blockieren eines nadellosen Injektors in Miniaturausführung zum Injizieren einer Flüssigkeit, die einen pharmazeutischen Wirkstoff enthält, in tierisches oder menschliches Gewebe.

## Claims

1. Apparatus (1) with blocking device which comprises a locking-stressing-mechanism with an operating spring and a spring transfer member in which is accommodated a piston which is mounted to be moveable in a cylinder, and these components are housed in a two-part housing which comprises an upper housing part (1) and a lower housing part (2), and the two parts are mounted to be rotatable relative to each other, and the operating spring is tensioned by means of a screw thrust gear by manually rotating the two housing parts relative to each other, and at the same time as the housing parts are rotated relative to each other a mechanical counter is actuated which comprises a threaded spindle (10) and a slider (9), and the threaded spindle is mounted in the wall of the lower housing part, and the slider is moved along the spindle by an amount which depends on the number of rotations of the two housing parts relative to each other, wherein
• a recess (4, 5) is provided in the outer wall of the lower housing part and in the inner wall of the upper housing part, and
• the two recesses are opposite each other when the two housing parts are in a given rotary position, and
• a moveable blocking element (7a, 7b) is provided which is located initially only in the recess in the lower housing part, and
• a push rod for moving the blocking element is provided which cooperates with the slider (9) on the spindle (10) of the counter.

2. Apparatus according to claim 1, wherein
• the push rod (8a, 8b) is mounted on the slider (9).

3. Apparatus according to claim 1, wherein
• the push rod (8a, 8b) is mounted on the blocking element (7a, 7b).

4. Apparatus according to claim 1, wherein
• the push rod (8a, 8b) is constructed as an extension of the spindle (10) of the counter and the spindle (10) is mounted to be axially moveable.

5. Apparatus according to claim 1, wherein
• the blocking element is moveable in the axial direction.

6. Apparatus according to claim 1, wherein
• the blocking element is a pre-stressed leaf spring (7a, 7b).

7. Apparatus according to claims 1 and 6, wherein
• the blocking element is a pre-stressed leaf spring (7a, 7b) with two legs and is preferably made of metal.

8. Use of the apparatus according to claim 1 for blocking a high pressure atomiser of miniaturised construction for atomising a liquid which contains a pharmaceutical active substance to produce an aerosol.

9. Use of the apparatus according to claim 1 for blocking a needleless injector of miniaturised construction for injecting a liquid which contains a pharmaceutical active substance into animal or human tissue.

## Revendications

1. Appareil (1) avec dispositif de blocage qui comprend un mécanisme tendeur à blocage avec un ressort de travail et un raccord en S, dans lequel est monté un piston qui est logé de manière mobile dans un cylindre, et ces composants sont logés dans un boîtier en deux parties qui comprend une partie supérieure (1) et une partie inférieure (2), et les deux parties sont logées de manière rotative l'une contre l'autre, et le ressort de travail est tendu à la main à l'aide d'un engrenage à visser et pousser par rotation des deux parties de boîtier l'une contre l'autre, et simultanément à la rotation des parties de boîtier l'une contre l'autre, un mécanisme de comptage mécanique est actionné, lequel comprend une broche filetée (10) et un cavalier (9) et la broche filetée est montée dans la paroi de la partie de boîtier inférieur, et le cavalier est déplacé d'une partie dépendant du nombre de rotations des deux parties de boîtier l'une contre l'autre sur la broche,
- dans la paroi extérieure de la partie de boîtier inférieure et dans la paroi intérieure de la partie de boîtier supérieure étant présent respectivement un évidement (4, 5) et
- les deux évidements se faisant face en cas de position de rotation prescrite des deux parties de boîtier et
- un élément de blocage (7a, 7b) mobile qui se trouve tout d'abord uniquement dans l'évidement dans la partie de boîtier inférieure étant présent et
- un coulisseau pour le déplacement de l'élément de blocage étant présent, lequel coopère avec le cavalier (9) sur la broche (10) du mécanisme de comptage.

2. Appareil selon la revendication 1,
- le coulisseau (8a, 8b) étant monté sur le cavalier (9).

3. Appareil selon la revendication 1,
- le coulisseau (8a, 8b) étant monté sur l'élément de blocage (7a, 7b).

4. Appareil selon la revendication 1,
- le coulisseau (8a, 8b) étant réalisé en prolongement de la broche (10) du mécanisme de comptage et la broche (10) étant logée de manière mobile axialement.

5. Appareil selon la revendication 1,
- l'élément de blocage étant mobile dans le sens axial.

6. Appareil selon la revendication 1,
- l'élément de blocage étant un ressort à lames (7a, 7b) précontraint.

7. Appareil selon les revendication 1 et 6,
- l'élément de blocage étant un ressort à lames (7a, 7b) précontraint avec deux branches et se composant de préférence de métal.

8. Utilisation de l'appareil selon la revendication 1 pour le blocage d'un pulvérisateur à haute pression dans une réalisation miniature pour la pulvérisation d'un liquide qui contient une substance active pharmaceutique, pour un aérosol.

9. Utilisation de l'appareil selon la revendication 1 pour le blocage d'un injecteur sans aiguille dans une réalisation miniature pour l'injection d'un liquide qui contient une substance active pharmaceutique, dans un tissu animal ou humain.
